# EUROPEAN PATENT SPECIFICATION

(11) **EP 3 518 853 B1**
(45) Date of publication and mention of the grant of the patent: **21.10.2020**
(21) Application number: 17791154.2
(22) Date of filing: 27.09.2017
(51) Int. Cl.: A61F 13/62, D04H 1/541, A44B 18/00, D04H 1/4374, D04H 1/559, D04H 11/08, B32B 5/26, D04H 1/54

(54) **LOOP MEMBER AND DIAPER**
SCHLAUFENELEMENT UND WINDEL
ÉLÉMENT FORMANT BOUCLE ET COUCHE

(30) Priority: 29.09.2016 JP 2016190790
(43) Date of publication of application: 07.08.2019
(73) Proprietor: 3M Innovative Properties Company, Saint Paul, Minnesota 55133-3427 (US)
(72) Inventor: MORISHITA, Kenichiro, Tokyo 141-8684 (JP)
(74) Representative: Vossius & Partner Patentanwälte Rechtsanwälte mbB
(86) International application number: PCT/IB2017/055909
(87) International publication number: WO 2018/060885

(56) References cited:
- EP-A1- 2 862 708
- WO-A1-92/20251
- US-A- 4 389 443
- US-A1- 2008 260 989

## Description

### Technical Field

The present invention relates to a loop member and a diaper.

### Background Art

Conventionally, surface fasteners have been widely used to secure or bind a variety of articles, including fiber products, plastic products, paper products, industrial articles, electronic components, construction materials, and the like. For example, sanitary articles (e.g. paper diapers, etc.) to which surface fasteners are attached as fastening members are known. Surface fasteners utilizing a variety of engagement methods are known, such as paired fasteners constituted by a male member having a hook-like engaging element and a female member capable of engaging with the engaging element. In particular, surface fastener fastening members made of nonwoven fabric have superior pliability and air permeability, making them advantageous for use in sanitary articles and the like.

Patent Document 1 discloses a nonwoven fabric having a recessed-and-protruded surface, constituted by a first fiber layer containing thermally shrunk fibers, on one or both faces of which is layered a second fiber layer containing non-contractile fibers, the nonwoven fabric being characterized in that the two fiber layers are partially integrated in the through-thickness direction at fused sections, and the thermal shrinkage of the first fiber layer causes the second fiber layer to protrude at a superficial section, thereby forming regular raised sections, between the fused sections.

Patent Document 2 discloses a loop material for a hook-and-loop fastener, the material being constituted by a composite nonwoven fabric; the material being characterized in that the composite nonwoven fabric is constituted by: a loop layer constituted by a carded web of unbonded thermoplastic crimped staple fibers, the staple fibers being 1.5 to 6.0 dTEX, and the carded web having a weight of 10 to 35 g/m²; a base layer over which the loop layer is facingly laid, the base layer being of a spun-melt nonwoven fabric having a weight of 5 to 30 g/m²; and a plurality of bonded regions joining the loop layer to the base layer; and the composite nonwoven fabric has an air permeability of 1500 L/m²/sec or less.

Patent Document 3 discloses a female part for a refastenable fastening device; the female part being characterized in being capable of engaging with a complementary hook fastening part including a base including individual hooks each of which projects outward from the base and has a dull head, and including a backing and a nonwoven web; the nonwoven web having a weight of 6 to 42 g/m² and being constituted by a plurality of fibers; the planar surface area occupied by bonds between the fibers of the nonwoven web being less than 10% of the total area of the nonwoven web; and the nonwoven web being bonded and affixed to the backing so that the total planar surface area occupied by both bonds between the fibers of the nonwoven web and bonds between the nonwoven web and the backing is 10% to 35% of the total area of the female part.
EP 2 862 708 A1 relates to a loop-forming closure element for hook-and-loop fasteners, comprising an embossed nonwoven material with continuous fibers and carded staple fibers. The continuous fibers and the staple fibers are entangled with one another and form a common nonwoven fabric layer of a fiber mixture.

### Citation List

### Patent Literature

Patent Document 1: JP-A-09-003755
Patent Document 2: JP-T-2010-524573
Patent Document 3: JP-T-6-507800
Patent Document 4: EP 2 862 708 A1

### SUMMARY OF THE INVENTION

The present invention is defined by the claims.

In surface fasteners, good engagement strength between the female member and the male member needs to be maintained even after the members have been repeatedly engaged and disengaged. In a surface fastener wherein a loop member utilizing nonwoven fabric is provided as a female member, repeated engagement and disengagement degrades the loop member, thereby reducing engagement strength. For example, an adult diaper is constituted by an outer member and an inner pad, the outer member including a surface fastener. In an adult diaper of this sort, the frequency with which the outer member is replaced is ordinarily comparatively low (for example, every three days), whereas the inner pad alone is replaced multiple times (for example, twenty or more times) every time the outer member is replaced. Since the surface fastener will be repeatedly engaged and disengaged multiple times, especially good engagement strength durability (i.e., maintenance of engagement strength after repeated engagement and disengagement) is demanded of the loop member of the surface fastener.

If the surface at which the loop member engages with the male member is constituted by a nonwoven fabric of staple fibers, suitably entangling the fibers making up the staple fiber nonwoven fabric so as to suppress shedding of the fibers is effective in improving engagement strength durability. However, a staple fiber nonwoven fabric of this short presents the problem of having low engagement strength. Low initial engagement strength (i.e., the engagement strength of the loop member when the loop member is first used), in particular, is undesirable in terms of the handling of the surface fastener. However, a loop member that strikes a balance between good initial engagement strength and good engagement strength durability has yet been proposed.

An object of the present invention is to provide a loop member capable of solving the problem described above and striking a balance between good initial engagement strength and engagement strength durability, as well as a diaper, especially preferably an adult diaper, including the same.

### Effect of the Invention

According to the present invention, there is provided a loop member capable of striking a balance between good initial engagement strength and engagement strength durability, as well as a diaper, especially preferably an adult diaper, including the same.

### Brief Description of the Drawings

FIG. 1 is a drawing illustrating a loop member according to one embodiment of the present invention.
FIG. 2 is a drawing illustrating a loop member according to one embodiment of the present invention.

### Description of Embodiments

Exemplary embodiments of the present invention will be described hereafter; however, the present invention is not limited to the following embodiments, and is intended to encompass various modifications within the scope of the claims. Unless otherwise noted, feature values set forth herein are as measured via the methods described in the examples section of the present invention, or methods that would be understood as being equivalent thereto by a person having ordinary skill in the art.

One embodiment of the present invention is:
a loop member for a surface fastener, the member including a first loop layer, a second loop layer, and a base layer disposed in that order,
the first loop layer, second loop layer, and base layer containing a staple fiber nonwoven fabric, and
the first loop layer and the second loop layer having different densities as defined in claim 1.

The loop member according to the present invention can be used as a loop member for forming a surface fastener based on various conventionally known fastening means. More specifically, the staple fiber nonwoven fabric contained in the first loop layer and the second loop layer (subsequent references simply to a "loop layer" are intended as collectively referring to both the first loop layer and the second loop layer) of the loop member according to the present invention is intended to function as a female member engaging element.

A hook is an example of a preferable male member inasmuch as it is capable of strong engagement with the nonwoven fabric engaging element. The hook is constituted by a projecting part projecting in the thickness direction of the surface fastener. The projecting part may be of any type as long as satisfactory engaging force can be obtained; preferable examples include mushroom-shaped, anchor-shaped, and letter-J-shaped. The density of projecting parts included in the hooks will typically be about 500 to 5000 per square inch. Examples of the materials of the hooks include polyethylene (e.g., high density polyethylene), polypropylene, polystyrene, polyvinyl chloride, polyethylene terephthalate, polybutylene terephthalate, nylon, polycarbonate, polymethyl methacrylate, polyacetal, polymethyl pentene, acrylonitrile-styrene-butadiene, polyphenylene ether, and polyphenylene sulfide; as well as styrene-based elastomer such as styrene-butadiene-styrene and styrene-isoprene-styrene; olefin-based elastomers such as ethylene-alpha-olefin copolymers; ester-based elastomers; amide-based elastomers; urethane-based elastomers; vinyl chloride-based elastomers; silicone-based elastomers; fluorine-based elastomers; and alloys thereof. The loop member according to the present invention may be part of a fastening member configured so that the hooks and the loop member, which contains a staple fiber nonwoven fabric as an engaging element with which the hooks can engage, are both present on the same surface.

In one embodiment, the loop member of the present invention can be used as a female member, and combined with a male member to constitute a surface fastener. In another embodiment, the surface fastener may be a pair of members in which, for example, both structures, i.e., a male member and a female member constituted by the loop member of the present invention, are present on the same surface.

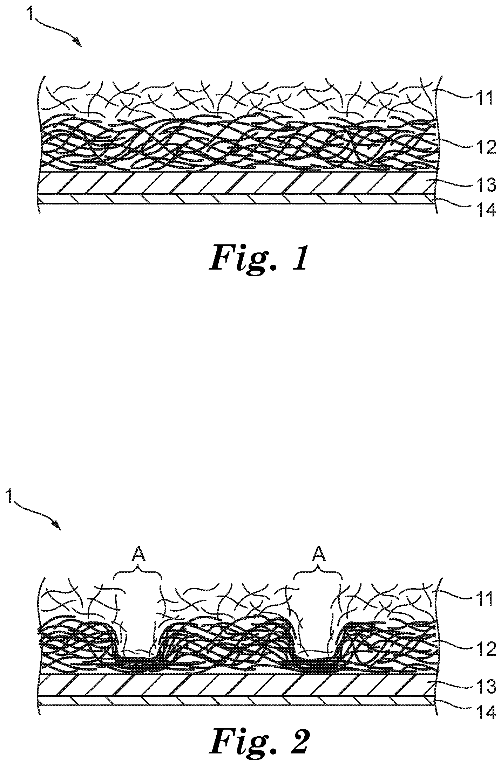

FIG. 1 is an illustration of an example of a loop member according to one embodiment of the present invention. In FIG. 1, a loop member 1 includes a second loop layer 12 and a base layer 13 disposed in that order on a first loop layer 11. In a typical embodiment, the loop member 1 can further include a printed layer 14 disposed on the base layer 13 (in FIG. 1, on the undersurface thereof).

The staple fiber nonwoven fabric contained in the first loop layer and the second loop layer (subsequent references simply to a "loop layer" are intended as collectively referring to both the first loop layer and the second loop layer) of the loop member according to the present invention is intended to function as an engaging element. The staple fiber nonwoven fabric contained in the base layer may constitute a printing surface.

The first loop layer, second loop layer, and base layer contain the staple fiber nonwoven fabric. In a typical embodiment, the loop member may be essentially constituted by nonwoven fabric and, more typically, may be essentially constituted by a staple fiber nonwoven fabric. Loop members essentially constituted by nonwoven fabric are capable of having pliability and air permeability suitable for use in sanitary articles. A staple fiber nonwoven fabric is advantageous, as such a fabric is capable of forming a thin, soft layer. As used herein, the term "staple fiber nonwoven fabric" refers to nonwoven fabric of which at least a main portion (more than 50 mass% of the constituent fibers) is constituted by staples (i.e., short fibers), in distinction to a nonwoven fabric constituted by filaments (i.e., long fibers). Staple fiber nonwoven fabrics include carded nonwoven fabrics, air-laid nonwoven fabrics, wet-laid nonwoven fabrics, and the like. Meanwhile, filament nonwoven fabrics generally include spunbond nonwoven fabrics and the like. The staples may generally have a length of up to a few hundred mm, though the present invention is not limited to such.

The loop member includes a first loop layer, a second loop layer, and a base layer, allowing the properties of the different layers to be independently controlled according to purpose. The inventors focused on the fact that, while it is vital that good engagement strength durability as manifested by engagement strength (especially disengagement strength) between a pair of fastening members (such as with the male members, also called hooks, if the loop member according to the present invention is used as a female member) be maintained even after repeated unfastening, designing a nonwoven fabric so as to ensure engagement strength durability makes it difficult to obtain extremely good engagement strength *per se.* In particular, the inventors focused on the fact that, inasmuch as the engagement strength at first use (i.e., initial engagement strength) greatly affects the handling of the fastening members, designing the fabric so as to yield increased initial engagement strength is effective in improving the handling of the fastening members. The inventors also focused on the fact that good printing properties (i.e., the ability to present a clearly printed design) are also required for fastening members, and that a high-smoothness printing surface is required in order to realize such properties. According to the loop member of the present invention, which includes a first loop layer and a second loop layer of different densities and a base layer, the contribution of the combination of the first and second loop layers allows a balance between initial engagement strength and engagement strength durability, and the contribution of the base layer allows good printing properties and member shape retention.

The density and pliability of the nonwoven fabric, while also affected by the material constituting the fibers making up the nonwoven fabric, are primarily affected by the morphological structure of the nonwoven fabric, which varies according to fiber diameter, the method by which the nonwoven fabric is formed, any treatments performed upon the formed nonwoven fabric, etc. A loop member according to one embodiment of the present invention includes a first loop layer and a second loop layer of different densities; this means that the morphological structure differs between the first loop layer and the second loop layer. A loop member according to one embodiment of the present invention includes a first loop layer and a second loop layer of different degrees of pliability; this means that the morphological structure differs between the first loop layer and the second loop layer. This difference in morphological structure allows differences between the first loop layer and the second loop layer in, for example, durability and strength of engagement to the other fastening member (typically, a male member) with which the loop member according to the present invention is combined. In a preferred embodiment, the density of the second loop layer is greater than the density of the first loop layer. In another preferred embodiment, the first loop layer is more pliable than the second loop layer.

Higher density in a nonwoven fabric tends to yield higher mechanical strength and lower porosity. The first loop layer contributes to the engagement strength of the loop member immediately following first use (i.e., initial engagement strength), while the second loop layer contributes to the engagement strength of the first loop layer following degradation after repeated engagement and disengagement (i.e., engagement strength durability). The combination of the first loop layer and the second loop layer, which is denser than the first loop layer, yields good initial engagement strength thanks to the contribution of the first loop layer, and acceptable engagement strength (i.e., less than the engagement strength yielded by the first loop layer, but sufficient for surface fastener use) on the part of the second loop layer after the first loop layer has degraded. Thus, such a combination is advantageous in striking a balance between good initial engagement strength and good engagement strength durability.

In a preferred embodiment, the density of the second loop layer is greater than the density of the first loop layer and less than the density of the base layer. A high-density base layer is advantageous for imparting a high-smoothness printing surface for obtaining good printing properties.

In a preferred embodiment, the staple fiber nonwoven fabric included in the first loop layer is adjusted to a comparatively low density by not being subjected to the thermal fusion, calendering, etc., described hereafter. As a result, the fabric is capable of imparting high initial engagement strength.

A nonwoven fabric of somewhat lower pliability, i.e., a somewhat harder fabric (i.e., having a high pliability value as measured according to the cantilever method described hereafter), tends to have greater mechanical strength and lower porosity. The first loop layer contributes to the engagement strength of the loop member immediately following first use (i.e., initial engagement strength), while the second loop layer contributes to the engagement strength of the first loop layer following degradation after repeated engagement and disengagement (i.e., engagement strength durability). The combination of the first loop layer and the second loop layer, which is denser than the first loop layer, yields good initial engagement strength thanks to the contribution of the first loop layer, and acceptable engagement strength (i.e., less than the engagement strength yielded by the first loop layer, but sufficient for surface fastener use) on the part of the second loop layer after the first loop layer has degraded. Thus, such a combination is advantageous in striking a balance between good initial engagement strength and good engagement strength durability.

In a preferred embodiment, the first loop layer is more pliable than the second loop layer. As a result, the fabric is capable of imparted high initial engagement strength.

The fibers in the second loop layer can be entangled via chemical bonding, an air-through process, or the like. Such processing is capable of increasing the density of the nonwoven fabric. In a preferred embodiment, the second loop layer contains a fused staple fiber nonwoven fabric. As used herein, the term "fused staple fiber nonwoven fabric" refers to staple fiber nonwoven fabric having a morphology in which the fibers making up the staple fiber nonwoven fabric are fixed together by melting the fibers. In performing state observation (e.g. state observation using an optical microscope), when melting marks of fiber material are found on the fiber surface of the staple fiber nonwoven fabric, and the fibers are bonded together at the sites of the melting marks, it can be confirmed that the staple fiber nonwoven fabric is fused. Here, the term "melting marks" refers to marks left by performing treatment intended to fuse only the loop layer (and thus only seen in the loop layer), as distinct from, for example, melting marks formed as a result of treatment for other purposes such as bonding the second loop layer to the base layer (such melting marks will be seen throughout both the second loop layer and the base layer). Fusion can be also confirmed by the staple fiber nonwoven fabric having a slightly rigid surface when touched by hand. In the present invention, the fusion by which the fused staple fiber nonwoven fabric is formed can be achieved, for example, through high-temperature air-through processing. As used herein, "high-temperature air-through processing" of the staple fiber nonwoven fabric refers to processing in which air of high temperature (at least higher than or equal to the melting point of the material on the outside of the fibers of the staple fiber nonwoven fabric) is passed through the staple fiber nonwoven fabric in the through-thickness direction thereof. Another conceivable method of fusion is to melt the outsides of the fibers using a heating means, chemical, etc., to affix the fibers together rather than passing heated air therethrough. High-temperature air-through processing is preferable from the perspective of fusing not only the one surface of the staple fiber nonwoven fabric, but also the inner surface or (the outsides of the fibers on) the opposite surface. High-temperature air-through processing is also preferable due to the use of chemicals or the like to melt the fibers often being eschewed in sanitary products such as diapers. Thus, the fibers are fused through a method other than methods that include applying a large amount of pressure directly to the staple fiber nonwoven fabric, such as rolling and the like. As a result, it is possible to improve durability while maintaining the engaging force of the loop member.

In a preferred embodiment, the base layer contains a calendered staple fiber nonwoven fabric. As used herein, the term "calendered staple fiber nonwoven fabric" refers to staple fiber nonwoven fabric that has a surface morphology that has been smoothed by the application of pressure. Accordingly, in the present invention, the calendering for forming the calendered staple fiber nonwoven fabric may encompass processing in which a layer to be processed is passed between a pair of smooth rollers, as well as processing in which a layer to be processed is smoothed by being passed between a smooth roller and a raised and recessed roller, for example. If, for example, a recessed and protruded roller is used, it is contemplated that a person skilled in the art will adjust the process for obtaining the desired smoothing effect (such as by passing the layer undergoing processing between the rollers multiple times) as appropriate. The "high-temperature air-through processing" and "calendering" differ, for example, in that the latter is a process of bringing the staple fiber nonwoven fabric directly into contact with a roller or the like for the purpose of smoothing, while the former is not.

In a preferred embodiment, the density of the first loop layer is about 10 kg/m³ or greater, about 15 kg/m³ or greater, or about 20 kg/m³ or greater from the perspective of obtaining good mechanical strength and initial engagement strength, and about 80 kg/m³ or less, about 60 kg/m³ or less, or about 40 kg/m³ or less from the perspective of obtaining good initial engagement strength by keeping the engaging element from becoming too dense, and obtaining good pliability.

In a preferred embodiment, the density of the second loop layer is about 30 kg/m³ or greater, about 40 kg/m³ or greater, or about 50 kg/m³ or greater from the perspective of obtaining good mechanical strength and engagement strength durability, and about 120 kg/m³ or less, about 100 kg/m³ or less, or about 80 kg/m³ or less from the perspective of obtaining good engagement strength by keeping the engaging element from becoming too dense, and obtaining good pliability.

In a preferred embodiment, the density of the base layer is about 100 kg/m³ or greater, about 120 kg/m³ or greater, or about 140 kg/m³ or greater from the perspective of obtaining good bonding between the loop layers and the base layer and obtaining good printing properties, and about 300 kg/m³ or less, about 250 kg/m³ or less, or about 200 kg/m³ or less from the perspective of obtaining good pliability.

As used herein, "density" refers to the value obtained by dividing basis weight by thickness. It is intended that the values measured for basis weight and thickness in the form of the loop member, or any of the values measured independently in the first loop layer, the second loop layer, and the base layer, , needs to be as disclosed herein. There will ordinarily be little difference between the basis weight values of the layers present in the loop member, and the basis weight values for the layers when individually present (i.e., prior to layering).

In a preferred embodiment, the basis weight of the first loop layer is about 1 gsm or greater, about 3 gsm or greater, or about 5 gsm or greater from the perspective of maintaining good mechanical strength and obtaining good initial engagement strength, and about 30 gsm or less, about 25 gsm or less, or about 20 gsm or less from the perspective of reducing costs and obtaining good pliability.

In a preferred embodiment, the basis weight of the second loop layer is preferably about 12 gsm or greater, about 15 gsm or greater, about 18 gsm or greater, or about 20 gsm or greater from the perspective of maintaining good mechanical strength and obtaining good engagement strength and engagement strength durability, and preferably about 50 gsm or less, about 45 gsm or less, or about 35 gsm or less from the perspective of obtaining a thin, inexpensive, pliable loop member.

In a preferred embodiment, the basis weight of the base layer is about 8 gsm or greater, about 10 gsm or greater, or about 12 gsm or greater from the perspective of maintaining good mechanical strength on the part of the base layer, and about 30 gsm or less, about 25 gsm or less, or about 20 gsm or less from the perspective of obtaining a thin, inexpensive, pliable loop member.

In a preferred embodiment, the thickness of the first loop layer is about 80 µm or greater, about 100 µm or greater, or about 120 µm or greater from the perspective of maintaining good mechanical strength and obtaining good initial engagement strength, and about 700 µm or less, about 600 µm or less, or about 500 µm or less from the perspective of reducing costs and obtaining good pliability.

In a preferred embodiment, the thickness of the second loop layer is preferably about 200 µm or greater, about 250 µm or greater, or about 300 µm or greater from the perspective of maintaining good mechanical strength and obtaining good engagement strength and engagement strength durability, and preferably about 1000 µm or less, about 750 µm or less, or about 500 µm or less from the perspective of reducing costs and obtaining good pliability.

In one embodiment of the present invention, the thickness of the base layer is about 15 µm or greater, preferably about 20 µm or greater, or about 25 µm or greater, from the perspectives of maintaining good mechanical strength and obtaining good engagement strength. From the perspective of cost reduction and pliability, the thickness is about 120 µm or less, preferably about 100 µm or less, or about 80 µm or less. With a base layer constituted of staple fiber nonwoven fabric (e.g. thermally bonded nonwoven fabric), in cases where the predetermined thickness is attained by calendering, both the reduction in the air permeance of the base layer to a desired level and the maintaining of the pliability can be achieved.

In the present invention, thickness can be measured using the loop member as a specimen according, for example, to the following method. First, a thickness measurement sample having an area of 10 mm x 10 mm is taken from the loop member, and the thickness thereof is measured. This thickness measuring is repeated five times at different locations of the sample, and the thickness results from the five measurements are taken as the thickness of the loop member. A thickness measuring instrument (ABSOKUTE KK-547-055, manufactured by Mitsutoyo, or comparable measuring instrument) is used to measure the thickness. The thickness of the loop member is measured by interposing the sample between the cylindrical end face and the base of the measuring instrument and, two seconds later, reading the digitally displayed thickness.

The respective proportions of the first loop layer, the second loop layer, and the base layer are determined from an image of the cross-sectional morphology (within a specific field of view) of the loop member obtained using a microscope (optical or scanning), and the proportions are used along with the thickness of the loop member determined as described above to calculate the thickness of each layer.

Alternatively, the layers may be separated from the loop member and individually measured for thickness. Specifically, to measure the loop layers, the base layer is removed from the measurement sample and the thickness of the loop layers when the loop layer is not bonded or affixed to the base layer is measured. To measure the base layer, the loop layers are removed from the measurement sample and the thickness of the base layer when the base layer is not bonded or affixed to the loop layer is measured.

In a preferred embodiment, the pliability of the first loop layer in the machine direction (MD) as measured according to the cantilever method defined in JIS L1096 is about 1 mm/25 mm width or greater, about 5 mm/25 mm width or greater, about 10 mm/25 mm width or greater, or about 15 mm/25 mm width or greater from the perspective of obtaining good mechanical strength and initial engagement strength, and about 40 mm/25 mm width or less, about 35 mm/25 mm width or less, or about 30 mm/25 mm width or less from the perspective of obtaining good pliability. The pliability in the cross direction (CD) is about 1 mm/25 mm width or greater, about 5 mm/25 mm width or greater, about 10 mm/25 mm width or greater, or about 15 mm/25 mm width or greater from the perspective of obtaining good mechanical strength and initial engagement strength, and about 40 mm/25 mm width or less, about 35 mm/25 mm width or less, or about 30 mm/25 mm width or less from the perspective of obtaining good pliability.

In a preferred embodiment, the pliability of the second loop layer in the MD as measured according to the cantilever method defined in JIS L1096 is about 20 mm/25 mm width or greater, about 40 mm/25 mm width or greater, or about 60 mm/25 mm width or greater from the perspective of obtaining good mechanical strength and engagement strength durability, and about 150 mm/25 mm width or less, about 130 mm/25 mm width or less, or about 110 mm/25 mm width or less from the perspective of obtaining good pliability. The pliability in the CD is about 20 mm/25 mm width or greater, about 30 mm/25 mm width or greater, or about 40 mm/25 mm width or greater from the perspective of obtaining good mechanical strength and engagement strength durability, and about 150 mm/25 mm width or less, about 120 mm/25 mm width or less, or about 90 mm/25 mm width or less from the perspective of obtaining good pliability.

As used herein, the term "pliability" refers to the degree of softness or rigidity. High pliability means that the material is highly flexible. A large numerical value as measured according to the cantilever method (as defined in JIS L1096), which is one typical measuring method used as a yardstick for pliability, indicates a low level of pliability.

In a preferred embodiment, the respective average linear densities of the first loop layer and the second loop layer are preferably about 2.0 den or greater or about 4.0 den or greater from the perspective of obtaining good engagement strength, and preferably about 15.0 den or less, about 12.0 den or less, about 10.0 den or less, about 8.0 den or less, or about 6.0 den or less from the perspective of maintaining good pliability on the part of the loop member.

In a preferred embodiment, the average linear density of the base layer is preferably about 0.5 den or greater, about 0.7 den or greater, about 0.9 den or greater, or about 1.0 den or greater from the perspective of maintaining good mechanical strength on the part of the base layer and ease of manufacturing, and preferably about 3.0 den or less, about 2.5 den or less, about 2.0 den or less, or about 1.5 den or less from the perspective of imparting a smooth printing surface and contributing to good printing properties.

The ratio (B/A) of the average linear density B of the fibers in the first loop layer to the average linear density A of the fibers in the base layer and the ratio (C/A) of the average linear density C of the fibers in the second loop layer to the average linear density A of the fibers in the base layer are about 1.5 or greater, preferably about 1.7 or greater, or about 2.0 or greater, from the perspective of striking a balance between engagement strength on the part of the loop layers and the printing properties of the base layer. Additionally, from the perspective of maintaining the pliability of the loop member, maintaining the mechanical strength of the base layer, and ease of manufacturing, the ratios are about 30 or less, preferably about 18 or less, or about 6 or less.

These ratios are calculated from values obtained by measuring the average linear density of each of the loop layers and the base layer using a linear density measuring device, namely Vibromat ME (manufactured by Textechno) or an equivalent measuring device. Specifically, first, about 10 strands of fiber are collected in as random a manner as possible. When collecting, broken fibers and otherwise damaged fibers cannot be used in the measuring, so such fibers are not collected. From the fibers that are collected, the end of one fiber is grabbed using tweezers and, without twisting or stretching the fiber, both ends of the fiber are secured in the clamp of the measuring instrument. Here, the thread is disposed so as to be vertical. At a set fiber length and tension, the fixed oscillation rate of the fiber secured in the clamp is measured from the oscillations thereof and converted to linear density. This operation is repeated five times, and the linear density average value of five strands of fiber is defined as the average linear density. The measurement environment is set to a temperature of 21°C±1°C and a humidity of 65%±2%. The ratio described above (average linear density of fibers in loop layer/average linear density of fibers in base layer) is calculated from the calculated average linear density of each of the loop layer and the base layer.

Examples of the fibers constituting the staple fiber nonwoven fabrics included in each of the loop layer and the base layer include polyolefins (e.g. polyethylene, polypropylene, and the like), polyester (e.g. PET, PBT, and the like), polyamide, polyurethane, EVA (ethylene-vinyl acetate), polylactic acid, rayon, fibers of copolymers and mixtures thereof, natural fibers, and the like.

In an aspect, from the perspective of preventing damage to the loop layer (the fiber falling out, or the like) due to engagement with the other fastening member, high-strength polyamide may be used for the loop layer. On the other hand, in consideration of material costs and environmental safety, polyethylene, polypropylene, polyester and the like are preferably used in the loop layer and/or the base layer.

The fibers constituting the staple fiber nonwoven fabric may be hydrophilic or hydrophobic. The fibers may also be composite fibers. Examples of preferable fiber forms of the composite fiber include sheath and core type (concentric type and eccentric type), parallel type (side-by-side type), split type (e.g. the cross-section is split into arc forms), and the like. Additionally, the fibers may be modified cross-section fibers, crimped fibers, heat-shrinkable fibers, or the like. These fibers may be used singly or in combinations of two or more kinds.

Additional examples include a two-component-type elastic composite fiber having a hard elastic component including crystalline polypropylene as a first component and having a thermoplastic elastomer as a second component; and mixed fibers including the two-component-type elastic composite fiber and other fibers.

In a preferred aspect, the fibers constituting the staple fiber nonwoven fabric included in the loop layers (especially the second loop layer) have a sheath-core structure. Additionally, in a preferred aspect, the fibers constituting the staple fiber nonwoven fabrics included in each of the loop layer and the base layer have a sheath-core structure. Sheath-core fibers that have a core with a first melting point (e.g. polyamide) and a sheath with a second melting point lower than the first melting point (e.g. polyethylene) are advantageous as the fibers that have a sheath-core structure because heat fusibility thereof is excellent. In consideration of material costs and environmental safety, and from the perspective that polyethylene, polypropylene, polyester and the like are preferably used in the loop layer and/or the base layer, examples of the sheath-core fibers that have a core with a first melting point and a sheath with a second melting point lower than the first melting point include sheath-core fibers that have a polypropylene core and a polyethylene sheath, sheath-core fibers that have a polypropylene core and a modified polyethylene sheath, sheath-core fibers that have a polypropylene core and a modified polypropylene sheath, and the like. In an aspect, from the perspectives of being light-weight, having high strength, having high pliability, and the like, sheath-core fibers that have a polypropylene core and a polyethylene sheath may be selected. Particularly, from the perspective of achieving excellent formation of the fusing sites, it is advantageous that the loop layer includes the staple fiber nonwoven fabric constituted of fibers that have the sheath-core structure described above.

For example, in a preferred aspect in which the fibers constituting the staple fiber nonwoven fabric included in the loop layers (especially the second loop layer) are sheath-core fibers with a core having a first melting point and a sheath having a second melting point, the first melting point is about 150°C or higher, about 160°C or higher, or about 170°C or higher from the perspective of obtaining good mechanical strength on the part of the loop layer, and, if polyethylene or polypropylene is used, about 200°C or lower in view of the properties of the material, and, if polyester is used, about 300°C or lower in view of the properties of the material. Additionally, in a preferred aspect, from the perspective of obtaining excellent mechanical strength of the loop layer, the perspective of achieving excellent formation of the affixitation sites by fusing in the loop layer, and the perspective of obtaining excellent pliability of the loop layer, the second melting point is about 130°C or lower, about 120°C or lower, or about 110°C or lower and, from the perspective that polymeric material is used, is about 80°C or higher or about 100°C or higher. A combination of the first melting point in the range described above and the second melting point in the range described above is particularly preferable. Note that, from the perspective of forming a staple fiber nonwoven fabric with excellent air permeability and smoothness, the first melting point and the second melting point being the ranges described above, and combinations thereof are advantageous for the base layer as well. Additionally, both cases where the core has a plurality of melting points and cases where the sheath has a plurality of melting points are possible. In these cases, all of the plurality of melting points of the core are the first melting point and all of the plurality of melting points of the sheath are the second melting point, but the lowest of each particularly contributes to the fusing and shape retention. In an aspect, an example of the first melting point of the core and the second melting point of the sheath in the present invention may be related to the lowest melting point of the first melting points of the core and the lowest melting point of the second melting points of the sheath. Note that the melting points are values measured by DSC or the like.

In another preferred aspect, the fibers constituting the staple fiber nonwoven fabric included in the loop layer may be fibers of a single material that has a melting point within the range described above as, for example, the first melting point.

The staple fiber nonwoven fabrics included in each of the loop layer and the base layer may be staple fiber nonwoven fabrics produced using a common method for producing staple fiber nonwoven fabric and, examples thereof include carded nonwoven fabrics, air-laid nonwoven fabrics, and the like. Here, in cases where filament nonwoven fabric produced by a spunbond method or a meltblown method is used as the base layer, it is necessary to use a high density nonwoven fabric in order to reduce the air permeability of the resulting base layer. When a base layer constituted by a filament nonwoven fabric is made thinner through calendering, reduced thickness will tend to result in the obtained base layer being more rigid. On the other hand, in cases where the base layer including the staple fiber nonwoven fabric is made thinner through calendering, it is easier to impart pliability and an appropriate degree of air permeability. The method of bonding the fibers of the staple fiber nonwoven fabric may be thermal bonding, chemical bonding, hydroentangling, needle punching, stitch bonding, steam jetting, or the like. In a preferred aspect, from the perspective of obtaining a thin layer with excellent pliability, the staple fiber nonwoven fabric included in the base layer is a thermally bonded nonwoven fabric. In a preferred aspect, the staple fiber nonwoven fabric included in the second loop layer is obtained by fusing the staple fiber nonwoven fabric via high-temperature air-through processing. In a preferred embodiment, the staple fiber nonwoven fabric included in the first loop layer, rather than being fused via high-temperature air-through processing, is preferably left as a thermally bonded nonwoven fabric, or subjected to low-temperature air-through processing.

In an exemplary embodiment, the staple fiber nonwoven fabric of the second loop layer is fused. As used herein, "fused" means that the fibers making up the staple fiber nonwoven fabric are bonded to each other at affixation sites as the result of the fibers melting (typically, the surfaces of the fibers melting). As a result, the staple fiber nonwoven fabric of the second loop layer has superior mechanical strength, and is thus capable of exhibiting good retention of engagement strength after repeated debonding.

The fusing can be performed using any device capable of high-temperature air-through processing. For example, an oven used in the production of the nonwoven fabric can be used as the device. However, in a typical aspect of the present invention, the fusing is performed at a temperature higher than normal temperature conditions set for each of the nonwoven fabric materials in the production of the nonwoven fabrics. Accordingly, in a typical aspect of the present invention, a device capable of air-through processing at a temperature such as that described later is used.

When the staple fiber nonwoven fabric of the aforementioned material is fused, a higher degree of melting of the fibers, brought about by setting the melt temperature to a higher temperature or the like, contributes to an increase in the affixitation sites between the fibers, formed by the melting of the fiber surface. When there are many affixitation sites between the fibers, gaps between the fibers in the staple fiber nonwoven fabric will decrease, and the volume of the staple fiber nonwoven fabric will decrease. In a typical aspect, in cases where the fusing is performed by high-temperature air-through processing, the fusing temperature (specifically the air temperature) and the air blow amount can be set in accordance with the desired degree of formation of the affixitation sites, according to the purpose. Hereinafter, an aspect of an example of the temperature and the air blow amount is described, but the present invention is not limited thereto.

In a preferred aspect in which the sheath is made of polyethylene, the staple fiber nonwoven fabric of the second loop layer is fused at a fusing temperature (e.g., the air temperature in the high-temperature air-through processing) of about 135°C to about 160°C. From the perspective of ensuring good formation of affixation sites, thereby keeping the staple fiber nonwoven fabric of the loop layers from shedding fibers, obtaining good mechanical strength, and obtaining good engagement strength retention, the fusing temperature is about 135°C or higher or about 140°C or higher, and, from the perspective of maintaining good pliability and air permeability on the part of the loop layers, about 160°C or lower, about 150°C or lower, or about 145°C or lower.

In an exemplary embodiment, the fibers making up the staple fiber nonwoven fabric included in the second loop layer are sheath-core fibers with a core having a first melting point and a sheath having a second melting point, and fused at a temperature between the first melting point and the second melting point.

In a preferred aspect, from the perspective of obtaining excellent formation of the affixitation sites, a difference (T1-T2) between a fusing temperature (T1) and a melting point (T2) of the material constituting the surface of the fibers to be crimped is about 5°C or higher, about 10°C or higher, or about 30°C or higher.

In a preferred aspect, from the perspective of ensuring good affixation site formation, thereby keeping the staple fiber nonwoven fabric of the loop layers from shedding fibers, and obtaining good mechanical strength and good retention of engagement strength, the air blow level in the high-temperature air-through processing is about 1% or greater, about 10% or greater, about 20% or greater, or about 30% or greater, and, from the perspective of preventing the affixation sites from becoming excessively numerous and maintaining excellent pliability and air permeability, about 100% or less or about 50% or less. Note that the air blow amount is selected so as to be balanced with the temperature.

In a preferred embodiment, the tensile strength at break of the second loop layer is about 20 N/50 mm or greater, about 25 N/50 mm or greater, or about 30 N/50 mm or greater from the perspective of obtaining good engagement strength on the part of the loop member, and about 200 N/50 mm or less or about 100 N/50 mm or less from the perspective of obtaining excellent pliability and air permeability on the part of the loop member.

In a preferred aspect, the loop layer is substantially not subjected to calendering for the purpose of surface smoothing. As a result, the staple fiber nonwoven fabric included in the loop layer can be caused to function excellently as the engaging element. Note that compression applied to the loop layer such as compression from the rolls or the like when bonding and/or fixing the loop layer and the base layer is not eliminated. For example, in cases where only one surface of the staple fiber nonwoven fabric layer is subjected to surface smoothing, sometimes a portion of the other surface of the staple fiber nonwoven fabric layer is subjected to surface smoothing at the same time. In these cases, the engagement strength (e.g., peel strength and shear strength) of the staple fiber nonwoven fabric present on the other surface may be reduced. In the case of the loop member of the present invention, by contrast, the loop layers are combined with the calendered base layer; thus, the loop layers are not affected by the calendering of the base layer. Accordingly, the loop layers can be formed without compromising the desired engagement strength.

The method and conditions of the calendering for obtaining the calendered staple fiber nonwoven fabric included in the base layer can be set according to the purpose thereof. If the density of the base layer increases due to the calendering, the base layer can have a smooth surface.

For example, the calendering is performed under conditions set so that the staple fiber nonwoven fabric for use in the base layer is compressed to a desired thickness. In an exemplary aspect in which the base layer is constituted by polypropylene/polyethylene sheath-core fibers, the calendering can be performed at a roll temperature of about 100°C to about 180°C.

The staple fiber nonwoven fabric of the calendered base layer is advantageous in that it contributes to the stable bonding of the base layer to the loop layers, that is, to the stable retention of the loop layers by the base layer, and increases the engagement strength of the loop member. Additionally, the calendered base layer can have a lower air permeance compared to nonwoven fabric that has not been calendered. As a result, excessively high air permeances that are disadvantageous in the production process can be avoided while maintaining the desired air permeance, which is a result of the contribution obtained by using nonwoven fabric.

In a typical aspect, the base layer may be calendered to the degree that the base layer becomes film-like (that is, a state where the smoothness of the nonwoven fabric surface is high and fine printing on the nonwoven fabric surface is possible). The film-like base layer that has been calendered has a lower air permeance compared to the base layer prior to the calendering.

The surface of the printed layer forming surface of the base layer may be subjected to surface treatment (e.g. corona discharge treatment, E-beam treatment). Additionally, the loop layer and/or the base layer may be subjected to coloring or other treatment.

From the perspective of excellently obtaining the advantages of the loop member of the present invention, the loop layer and/or the base layer are typically constituted from a staple fiber nonwoven fabric. Additional layers other than staple fiber nonwoven fabric may also be included. The staple fiber nonwoven fabric included in the first loop layer, the second loop layer, and the base layer may be single- or multiple-layered. Examples of additional layers include adhesive layers, resin films, knitted fabric, woven fabric, paper, laminates thereof, and the like. The method for forming the additional layers is not particularly limited, and any conventionally known method such as coating, dry lamination, extrusion lamination, wet lamination, thermal lamination, ultrasonic method, may be used.

In an exemplary aspect, the loop member further includes a printed layer. The printed layer may be fixed directly on the base layer. In the present invention, the printed layer being fixed directly on the base layer means that the printed layer is disposed on (that is, is in contact with) the base layer without any other members or layers disposed therebetween, and that the printed layer is not substantially peelable from the base layer while maintaining the form of the printed layer. Such a printed layer is advantageous because a thin loop member can be obtained through a simple process.

The printed layer includes at least an ink layer, and is either a single layer or is a plurality of layers. The printed layer may be constituted only of the ink layer or, in addition to the ink layer, may include a base coat and/or a top coat. The base coat and the top coat each contribute to the improvement of the fixibility of the ink layer on the base layer.

The ink layer and the optional base coat and top coat each may exist as a continuous layer on the base layer or may be disposed discontinuously; and may be appropriately designed depending on a desired purpose such as, for example, the intended design of the ink layer. Any design may be selected, including characters, drawings, patterns, or the like.

Examples of the material of the ink layer include various types of conventionally known inks, and water-soluble and solvent-based inks may be used. Resins normally used in the art may be used as the resin included in the ink. Examples of such resins include acrylic resins, polyurethane resins, polyamide resins, urea resins, polyester resins, vinyl chloride resins, vinylidene chloride resins, vinyl chloride-vinyl acetate copolymer resins, ethylene-vinyl acetate copolymer resins, olefin resins, chlorinated olefin resins, epoxy resins, petroleum-based resins, cellulose derivative resins, and the like. If the ink layer has excellent toughness, the ink layer will not be prone to becoming defective, even in cases where the ink layer is exposed when an article that is provided with the loop member of the present invention is used. From this perspective, acrylic-based inks, urethane-based inks and the like, for example, are preferable.

From the perspective of the fixibility of the printed layer on the base layer, it is preferable that the ink layer itself be adhesive. Examples of the material for forming the adhesive ink layer include materials obtained by mixing ink with the material used for the adhesive layer described above.

The thickness of the ink layer and any optional base coat and top coat making up the printed layer are, for example, about 0.5 µm or greater and about 20 µm or less, about 1 µm or greater and about 15 µm or less, or about 2 µm or greater and about 10 µm or less, respectively (although not limited thereto), from the perspective of the robustness of the printed layer and texture (pliability, air permeability, etc.) when the member is used in a sanitary product.

In an aspect, the printed layer may be adhered to the base layer by an adhesive. In this case, the fixibility of the printed layer is excellent. Examples of the material of the adhesive include adhesive polymers such as acrylic polymers (e.g., SK dyne (an acrylic adhesive available from Soken Chemical & Engineering Co., Ltd.)), silicone-based polymers, and rubber-based polymers; and hotmelt adhesives such as Jet-melt (trade name) EC-3748 (available from 3M) and the like. Furthermore, optionally, tackifying resins, crosslinking agents, or other additives may be combined with the adhesive polymers described above.

The thickness of the adhesive can ordinarily be from about 5 µm to about 200 µm. The adhesive is formed, for example, by applying the material of the adhesive described above on the surface of the base layer and, thereafter, drying.

Various production methods are possible for the loop member of the present invention. In an exemplary aspect, the loop member can be produced by a method including: preparing the staple fiber nonwoven fabric for the loop layer and the staple fiber nonwoven fabric for the base layer; subjecting the staple fiber nonwoven fabric of the base layer to calendering; fusing the staple fiber nonwoven fabric of the loop layer; layering the calendered staple fiber nonwoven fabric and the fused staple fiber nonwoven fabric for the loop layer; bonding these two layers together so as to obtain a laminated web having the base layer and the loop layer; and optionally forming the printed layer on the base layer side of the laminated web.

The fabrication of the staple fiber nonwoven fabrics, the calendering of the base layer, and the fusing of the loop layer can, for example, be performed via the method and conditions described above. Next, the calendered staple fiber nonwoven fabric for use in the base layer, the staple fiber nonwoven fabric for use in the first loop layer, and the fused staple fiber nonwoven fabric for use in the second loop layer are layered.

In a typical embodiment, the loop layers and the base layer are bonded to each other via embossing, point bonding (thermal bonding), chemical bonding, needle punching, water jets, air-through processing, or the like. FIG. 2 is a drawing illustrating an example of the loop member according to an aspect of the present invention, and depicts a state in which the loop layer and the base layer are secured by embossing. As illustrated in FIG. 2, the first loop layer 11, the second loop layer 12, and the base layer 13 of the loop member 1 can be bonded to each other in an embossed pattern A. The shape of the emboss pattern formed through the embossing is not particularly limited, and may be rectangular, a wave-shape, or the like. For example, in an illustrated aspect of bonding regions where the loop layer and the base layer are bonded, in cases where the loop layer and/or the base layer include carded nonwoven fabric, a pitch of the bonding regions in a fiber direction of the carded nonwoven fabric is preferably disposed so as to be shorter than a pitch of the bonding regions in a direction substantially orthogonal to the fiber direction. Such bonding regions are advantageous because fluffing of the carded nonwoven fabric can be avoided. The loop layer is configured to bond to the calendered base layer and, thus, it is possible to securely and strongly fix the loop layer to the base layer. This prevents problems such as debonding of the loop layers from the base layer and tearing, breakage, etc., of the loop layers from occurring when the loop member is disengaged from another fastening member (such as a male member). Conditions of the embossing may be set to, for example, a temperature of about 110°C to about 180°C and a pressure in a range from 0 N/m² to about 1000 N/m².

Examples of methods for forming the printed layer on the base layer include a method in which the base layer is directly coated with a material for forming the printed layer (also referred to as a "printing material" in the present invention). Additionally, a method can be used in which a printed layer that has been preformed on a liner is transferred to the base layer, and then the liner is removed. The transfer method is preferable because the ink of the printed layer will not easily penetrate the base layer and the loop layer.

Specifically, in the transfer method, first, a printed layer is formed on a liner. A surface of the liner preferably has releasing properties sufficient to enable the transfer of the printed layer to the base layer. Various types of sheets that are conventionally known as transfer liners can be used as the liner, and examples thereof include silicone-coated kraft paper, silicone-coated polyethylene paper, silicone coated or uncoated polymeric material (e.g., polyethylene, polypropylene, or the like), as well as base materials coated with polymeric release agents such as silicone urea, urethanes, long chain alkyl acrylates, and the like. Suitable commercially available release liners include those sold under the product name "POLYSLIK" available from Rexam Release of Oakbrook, Illinois, and those sold under the name "EXHERE" available from Galtfelter Company of Spring Grove, Pennsylvania. The printed layer forming surface of the liner may be subjected to, for example, embossing or the like.

The printing material is applied on the liner described above. In a typical aspect, the ink is applied on the liner via a desired printing method such as, for example, roll coating, gravure coating, curtain coating, spray coating, screen printing, or the like. Alternatively, a top coat, the ink, and a base coat may be applied on the liner, in this order. According to the procedure described above, a transfer sheet in which the printed layer is formed on the liner is obtained.

Next, the printed layer is transferred from the liner to the base layer by passing the transfer sheet and the laminated web through a pair of rollers so that the printed layer side of the transfer sheet and the base layer side of the laminated web face each other. Thereby, a loop member in which the printed layer is formed on the base layer can be obtained.

Note that, in cases where the printed layer includes the ink layer, and a base coat and/or a top coat, the printed layer may be sequentially formed through a plurality of steps. For example, in the case of a printed layer including a base coat, an ink layer, and a top coat, the base coat may be formed beforehand on the base layer, then the ink layer may be transferred, and then the top coat may be formed. The printed layer can be directly fixed to the base layer through this method.

In a preferred embodiment, the basis weight of the loop member is preferably about 20 gsm or greater, about 25 gsm or greater, about 28 gsm or greater, or about 33 gsm or greater from the perspective of maintaining the mechanical strength of the loop member and obtaining good engagement strength, and preferably about 60 gsm or less, about 50 gsm or less, about 43 gsm or less, or about 37 gsm or less from the perspective of obtaining a thin, inexpensive, pliable loop member.

In a preferred embodiment, the 90° peel strength of the loop member between the other fastening member (specifically, surface fastener hooks constituting the male member) with which the loop member is engaged, as measured according to JTM-1221 using a 1600 pin/square inch hook member (1600 DH; manufactured by 3M) as a male member, is preferably about 2.5 N/25.4 mm or greater, about 2.7 N/25.4 mm or greater, about 2.9 N/25.4 mm or greater, or about 3.1 N/25.4 mm or greater from the perspective of good engagement strength, and preferably about 10 N/25.4 mm or less, about 8 N/25.4 mm or less, or about 6 N/25.4 mm or less from the perspective of good handling.

In a preferred embodiment, the 90° peel strength of the loop member between the other fastening member (specifically, surface fastener hooks constituting the male member) with which the loop member is engaged, as measured according to JTM-1221 using a 1600 pin/square inch hook member (1600 DH; manufactured by 3M) as a male member, on the tenth repetition after repeatedly engaging and disengaging the two ten times at 90° is preferably about 1.0 N/25.4 mm or greater, about 1.2 N/25.4 mm or greater, about 1.4 N/25.4 mm or greater, or about 1.6 N/25.4 mm or greater from the perspective of good engagement strength, and preferably about 10 N/25.4 mm or less, about 8 N/25.4 mm or less, or about 6 N/25.4 mm or less from the perspective of good handling.

In a preferred embodiment, the 135° peel strength of the loop member between the other fastening member (specifically, surface fastener hooks constituting the male member) with which the loop member is engaged, as measured according to JTM-1343 using a 1600 pin/square inch hook member (1600 DH; manufactured by 3M) as a male member, is preferably about 0.8 N/25.4 mm or greater, about 0.9 N/25.4 mm or greater, or about 1.0 N/25.4 mm or greater from the perspective of good engagement strength, and preferably about 10 N/25.4 mm or less, about 8 N/25.4 mm or less, or about 6 N/25.4 mm or less from the perspective of good handling.

In a preferred embodiment, the 135° peel strength of the loop member between the other fastening member (specifically, surface fastener hooks constituting the male member) with which the loop member is engaged, as measured according to JTM-1343 using a 1600 pin/square inch hook member (1600DH; manufactured by 3M) as a male member, on the tenth repetition after repeatedly engaging and disengaging the two ten times at 135° is preferably about 0.4 N/25.4 mm or greater, about 0.5 N/25.4 mm or greater, or about 0.55 N/25.4 mm or greater from the perspective of good engagement strength, and preferably about 10 N/25.4 mm or less, about 8 N/25.4 mm or less, or about 6 N/25.4 mm or less from the perspective of good handling.

The loop member of the present invention can be used for various articles such as, for example, for the fixing of various types of applicable subjects to floors, walls, clothing, cleaning members, automobile interior materials, and the like. The loop member according to the present invention, by virtue of its configuration, is capable of having good pliability and air permeability, as well as good engagement strength and good retention thereof after repeated disengagement, and is thus advantageous as a loop member for use in a surface fastener attached to sanitary products, especially diapers (especially preferably adult diapers).

In another embodiment of the present invention, a diaper is provided that includes a surface fastener including the loop member according to the embodiment of the present invention described above.

Examples of sanitary articles include child and adult diapers, napkins for sanitary and other uses, and the like; due to the good engagement strength durability thereof (i.e., retention of engagement strength after repeated disengagement), the loop member according to the present invention is particularly suited to adult diapers, which are frequently taken off. In a typical aspect, the loop member according to the present invention can be combined with a loop member according to the present invention or any other fastening member and used as a surface fastener of a sanitary article, preferably a diaper, especially preferably an adult diaper.

In a preferred aspect, the sanitary article has excellent air permeability. More specifically, the air permeability of the sanitary article as measured according to the Gurley method is preferably about 5 sec or less, about 3 sec or less, or about 1 sec or less from the perspective of being capable of imparting the sanitary article with a good level of comfortability when it is worn. Though there is no particular limitation upon the minimum air permeability, air permeability is about 0.1 sec or greater in certain embodiments.

A production method of the sanitary article is not particularly limited, and an example of a method is described below. Any conventionally known constituent can be used in addition to the loop member in the sanitary article, and specific description thereof is omitted herein. In the sanitary article, the method for attaching the loop member to the application section may be any conventionally known method. The printed layer forming surface of the loop member is bonded to the application section using a known bonding method (gluing, thermal fusion, bonding by ultrasonic machining or the like, mechanical anchoring via sewing or stapling, etc.). For anchoring by means of gluing, known adhesives such as rubber based adhesives such as SIS and SBS, acryl based adhesives, silicone based adhesives, EVA based adhesives, and the like may be suitably selected as required, although the adhesive is not limited to these resins.

### Examples

The present invention will now be described in further detail using examples, but the present invention is not limited to these examples.

### Preparation of loop member

### Examples 1 to 4

### Comparative Example 1

The staple fiber nonwoven fabrics used were as follows. The staple fiber nonwoven fabrics described hereafter are all constituted by sheath-core fibers of polyethylene (sheath; melting point: about 115°C) and polypropylene (core; melting point: about 163°C).

### Example 1

First loop layer: Prod. No. 6.6 ESC cure repeat PE1185; average linear density: 6 den; fiber length: 40 mm; commercially available from Fibervisions
Second loop layer: Prod. No. 6.6 ESC cure repeat PE1185; average linear density: 6 den; fiber length: 40 mm; commercially available from Fibervisions
Base layer: Prod. No. 1.7 ESC cure repeat PE1185; average linear density: 1.5 den; fiber length: 40 mm; commercially available from Fibervisions

### Example 2

First loop layer: average linear density: 4 den; fiber length: 40 mm
Second loop layer: Prod. No. 6.6 ESC cure repeat PE1185; average linear density: 6 den; fiber length: 40 mm; commercially available from Fibervisions
Base layer: Prod. No. 1.7 ESC cure repeat PE1185; average linear density: 1.5 den; fiber length: 40 mm; commercially available from Fibervisions

### Example 3

First loop layer: Prod. No. 6.6 ESC cure repeat PE1185; average linear density: 6 den; fiber length: 40 mm; commercially available from Fibervisions
Second loop layer: Prod. No. 6.6 ESC cure repeat PE1185; fiber length: 40 mm; commercially available from Fibervisions
Base layer: Prod. No. 1.7 ESC cure repeat PE1185; average linear density: 1.5 den; fiber length: 40 mm; commercially available from Fibervisions

### Example 4

First loop layer: average linear density: 4 den; fiber length: 40 mm
Second loop layer: 6.6 ESC cure repeat PE1185; average linear density: 6 den; fiber length: 40 mm; commercially available from Fibervisions
Base layer: 1.7 ESC cure repeat PE1185; average linear density: 1.5 den; fiber length: 40 mm; commercially available from Fibervisions

### Comparative Example 1

Loop layer (equivalent to second loop layer): Prod. No. 6.6 ESC cure repeat PE1185; average linear density: 6 den; fiber length: 40 mm; commercially available from Fibervisions
Base layer: Prod. No. 1.7 ESC cure repeat PE1185; average linear density: 1.5 den; fiber length: 40 mm; commercially available from Fibervisions

The staple fiber nonwoven fabrics for the first loop layer (not present in Comparative Example 1), the second loop layer, and the base layer were subjected to the treatments (point bonding, high-temperature air-through processing, low-temperature air-through processing, calendering) listed in Table 1, then pattern-embossed and layered at a temperature of about 135°C and a nip pressure of 80 bar (80 MPa) to obtain a loop member. The above-described processes were performed under the conditions described hereafter.

Point bonding (also referred to as thermal bonding) Pointing bonding was performed by passing the nonwoven fabrics through 120°C thermal point bonding rollers at a pressure of 20 MPa and a speed of 70 mpm. The thermocompression-bonded parts constituted 25% of the whole. Unlike calendering, point bonding does not involve thermocompressing substantially the entire surface for the sake of smoothing, but only partial thermocompression.

### High-temperature air-through processing

The nonwoven fabrics were fused via high-temperature air-through processing in the following oven under the following conditions.
Oven: STRAHM HiPer (trade name) Therm System (manufactured by Strahm Textile Systems AG)
Line speed: 70 mpm
Air-through temperature: 145°C
Air blow level(*): 30%
(*) against 100% of the maximum air blowing capacity of the oven.

### (Low-temperature) air-through processing

The nonwoven fabrics were fused via low-temperature air-through processing in the following oven under the following conditions.

Oven: STRAHM HiPer (trade name) Therm System (manufactured by Strahm Textile Systems AG)
Line speed: 70 mpm
Air-through temperature: 131°C
Air blow level(*): 23%
(*) against 100% of the maximum air blowing capacity of the oven.

### Calendering

100% calendering (football pattern) was performed by passing the nonwoven fabrics through 150°C thermal point bonding rollers.

### Evaluation of properties

### 1. 135°C peel strength

135° peel strength was measured according to JTM-1343 using a 1600 pin/square inch hook member (1600DH; manufactured by 3M Company; 20 mm × 25 mm) as a male member. The loop member and male member were engaged and peeled at 135°C ten times, and initial 135°C peel strength and the strength after the tenth repetition were recorded.

### 2. Pliability

The pliability of samples constituted by the materials used for the first and second loop layers prior to being layered into a loop member was measured according to the cantilever method defined in JIS L1096. A 25 mm by 250 mm test strip was taken from the samples. A first edge of the test strip was aligned with and placed on the front end of the platform of a cantilever-type testing machine. The 0 point of the steel ruler was adjusted to the mark D and, in this state, the steel ruler was placed on the test strip. The steel ruler and the test strip were pressed gently together at a constant speed in the direction of the inclined face. The steel ruler was moved and the test piece was allowed to rest for eight seconds, after which the length of the protruding test strip was read from the steel ruler. Measurement was performed in the machine direction (MD) and cross direction (CD) of the materials of each layer.

**Table 1**

| | | Comparative Example 1 | | Example 1 | | | Example 2 | | | Example 3 | | | Example 4 | | |
|---|---|---|---|---|---|---|---|---|---|---|---|---|---|---|---|
| Layer structure | | Second loop layer | Base layer | First loop layer | Second loop layer | Base layer | First loop layer | Second loop layer | Base layer | First loop layer | Second loop layer | Base layer | First loop layer | Second loop layer | Base layer |
| Treatment | | High-temperature air-through processing | Calendering | Low-temperature air-through processing | High-temperature air-through processing | Calendering | Low-temperature air-through processing | High-temperature air-through processing | Calendering | Point bonding | High-temperature air-through processing | Calendering | Point bonding | High-temperature air-through processing | Calendering |
| Average linear density (den) | | 6 | 1.5 | 6 | 6 | 1.5 | 4 | 6 | 1.5 | 6 | 6 | 1.5 | 4 | 6 | 1.5 |
| Average linear density ratio (to base layer) | | 4 | - | 4 | 4 | - | 2.7 | 4 | - | 4 | 4 | - | 2.7 | 4 | - |
| Basis weight (gsm) | | 30 | 15 | 10 | 25 | 10 | 10 | 25 | 10 | 10 | 25 | 10 | 10 | 25 | 10 |
| Thickness (µm) | 1 | 360 | 77 | 440 | 470 | 51 | 228 | 460 | 64 | 240 | 477 | 60 | 190 | 290 | 52 |
| | 2 | 580 | 80 | 450 | 406 | 60 | 271 | 390 | 65 | 302 | 421 | 64 | 137 | 396 | 60 |
| | 3 | 450 | 76 | 560 | 460 | 64 | 360 | 320 | 52 | 246 | 460 | 51 | 142 | 374 | 52 |
| | Average | 463 | 78 | 483 | 445 | 58 | 286 | 390 | 60 | 263 | 453 | 58 | 156 | 353 | 55 |
| Density (kg/m³) | | 65 | 193 | 21 | 56 | 171 | 35 | 64 | 166 | 38 | 55 | 171 | 64 | 71 | 183 |
| 135° peeling, first time (N/25.4 mm) | | 0.2 | | 0.8 | | | 0.6 | | | 0.5 | | | 0.4 | | |
| 135° peeling, tenth time (N/25.4 mm) | | 0.3 | | 0.5 | | | 0.5 | | | | | | | | |
| MD pliability (cantilever method) (mm/25 mm width) | | 96 | | 21 | 86 | | 24 | 86 | | 24 | 86 | | 21 | 86 | |
| CD pliability (cantilever method) (mm/25 mm width) | | 60 | | 20 | 55 | | 23 | 55 | | 23 | 55 | | 19 | 55 | |

The loop members according to the examples all exhibited good peel strength at the first and tenth repetitions. In Examples 1 to 4, the members exhibited good peel strength (i.e., good engagement strength durability) after ten repeated disengagements. Meanwhile, Comparative Example 1, which used a loop member of nonwoven fabric, had low first-repetition (i.e., initial) peel strength due to the lack of a first loop layer.

### Industrial Applicability

The loop member according to the present invention can be advantageously used in a diaper, especially an adult diaper.

### Reference Signs List

- 1: Loop member
- 11: First loop layer
- 12: Second loop layer
- 13: Base layer
- 14: Printed layer

## Claims

1. A loop member (1) for a surface fastener, the member (1) comprising a first loop layer (11), a second loop layer (12), and a base layer (13) disposed in that order,
the first loop layer (11), the second loop layer (12), and the base layer (13) containing a staple fiber nonwoven fabric, and
the first loop layer (11) and the second loop layer (12) having different densities, wherein the second loop layer (12) has a density that is greater than the density of the first loop layer (11) and less than the density of the base layer (13).

2. The loop member according to claim 1, wherein
the first loop layer (11) has a density of 10 kg/m³ to 80 kg/m³,
the second loop layer (12) has a density of 30 kg/m³ to 120 kg/m³, and
the base layer (13) has a density of 100 kg/m³ to 300 kg/m³.

3. The loop member according to claim 1,wherein the first loop layer (11) and the second loop layer (12) have different degrees of pliability.

4. The loop member according to claim 3, wherein the first loop layer (11) is more pliable than the second loop layer (12).

5. The loop member according to claim 4, wherein
the first loop layer (11) has a pliability as measured according to a cantilever method JIS L1096 of 1 mm/25 mm width to 40 mm/25 mm width, and
the second loop layer (12) has a pliability as measured according to a cantilever method of 20 mm/25 mm width to 150 mm/25 mm width.

6. The loop member according to any one of claims 1 to 5, wherein
the ratio (B/A) of the average linear density B of the fibers of the first loop layer (11) to the average linear density A of the fibers in the base layer is from 1.5 to 30, and
the ratio (C/A) of the average linear density C of the fibers of the first loop layer (11) to the average linear density A of the fibers in the base layer is from 1.5 to 30

7. The loop member according to any one of claims 1 to 6, wherein
the first loop layer (11) has a thickness of 80 µm to 700 µm,
the second loop layer (12) has a thickness of 200 µm to 1000 µm, and
the base layer (13) has a thickness of 15 µm to 120 µm.

8. The loop member according to any one of claims 1 to 7, wherein the staple fiber nonwoven fabric of the second loop layer (12) is fused, and the staple fiber nonwoven fabric of the base layer (13) is calendered.

9. The loop member according to any one of claims 1 to 8, wherein the staple fiber nonwoven fabric of the second loop layer (12) includes sheath-core fibers comprising a core having a first melting point and a sheath having a second melting point lower than the first melting point.

10. The loop member according to claim 9, wherein the staple fiber nonwoven fabric of the second loop layer (12) is fused in a temperature range between the first melting point and the second melting point.

11. The loop member according to any one of claims 1 to 10, wherein the staple fiber nonwoven fabric of the first loop layer (11), the second loop layer (12), and the base layer (13) each include sheath-core fibers comprising polypropylene cores and polyethylene sheaths.

12. The loop member according to any one of claims 1 to 11, wherein the 135° peel strength measured according to JTM-1343 of the loop member (1) after one repetition of the loop member (1) being engaged with and disengaged from surface fastener hooks comprising projecting parts and being capable of engagement with the loop member (1) is not less than 0.8 N/25.4 mm and not greater than 10 N/25.4 mm, and the 135° peel strength of the loop member after ten repetitions is not less than 0.4 N/25.4 mm and not greater than 10 N/25.4 mm.

13. A diaper comprising the loop member (1) described in any one of claims 1 to 12.

## Patentansprüche

1. Ein Schlaufenelement (1) für ein Flächenbefestigungsmittel, wobei das Element (1) eine erste Schlaufenschicht (11), eine zweite Schlaufenschicht (12) und eine Basisschicht (13) aufweist, die in dieser Reihenfolge angeordnet sind,
wobei die erste Schlaufenschicht (11), die zweite Schlaufenschicht (12) und die Basisschicht (13) einen Stapelfaservliesstoff enthalten, und
wobei die erste Schlaufenschicht (11) und die zweite Schlaufenschicht (12) unterschiedliche Dichten aufweisen, wobei die zweite Schlaufenschicht (12) eine Dichte aufweist, die größer als die Dichte der ersten Schlaufenschicht (11) und kleiner als die Dichte der Basisschicht (13) ist.

2. Das Schlaufenelement nach Anspruch 1, wobei
die erste Schlaufenschicht (11) eine Dichte von 10 kg/m³ bis 80 kg/m³ aufweist,
die zweite Schlaufenschicht (12) eine Dichte von 30 kg/m³ bis 120 kg/m³ aufweist und die Basisschicht (13) eine Dichte von 100 kg/m³ bis 300 kg/m³ aufweist.

3. Das Schlaufenelement nach Anspruch 1, wobei die erste Schlaufenschicht (11) und die zweite Schlaufenschicht (12) unterschiedliche Grade an Biegsamkeit aufweisen.

4. Das Schlaufenelement nach Anspruch 3, wobei die erste Schlaufenschicht (11) biegsamer als die zweite Schlaufenschicht (12) ist.

5. Das Schlaufenelement nach Anspruch 4, wobei
die erste Schlaufenschicht (11) eine Biegsamkeit, gemessen nach einem Cantilever-Verfahren JIS L1096, von 1 mm/25 mm Breite bis 40 mm/25 mm Breite aufweist, und
die zweite Schlaufenschicht (12) eine Biegsamkeit, gemessen nach einem Cantilever-Verfahren, von 20 mm/25 mm Breite bis 150 mm/25 mm Breite aufweist.

6. Das Schlaufenelement nach einem der Ansprüche 1 bis 5, wobei
das Verhältnis (B/A) der durchschnittlichen linearen Dichte B der Fasern der ersten Schlaufenschicht (11) zu der durchschnittlichen linearen Dichte A der Fasern in der Basisschicht 1,5 bis 30 beträgt, und
das Verhältnis (C/A) der durchschnittlichen linearen Dichte C der Fasern der ersten Schlaufenschicht (11) zu der durchschnittlichen linearen Dichte A der Fasern in der Basisschicht 1,5 bis 30 beträgt.

7. Das Schlaufenelement nach einem der Ansprüche 1 bis 6, wobei
die erste Schlaufenschicht (11) eine Dicke von 80 µm bis 700 µm aufweist,
die zweite Schlaufenschicht (12) eine Dicke von 200 µm bis 1000 µm aufweist, und
die Basisschicht (13) eine Dicke von 15 µm bis 120 µm aufweist.

8. Das Schlaufenelement nach einem der Ansprüche 1 bis 7, wobei der Stapelfaservliesstoff der zweiten Schlaufenschicht (12) verschmolzen ist und der Stapelfaservliesstoff der Basisschicht (13) kalandriert ist.

9. Das Schlaufenelement nach einem der Ansprüche 1 bis 8, wobei der Stapelfaservliesstoff der zweiten Schlaufenschicht (12) Mantel-Kern-Fasern aufweist, die einen Kern mit einem ersten Schmelzpunkt und einen Mantel mit einem zweiten Schmelzpunkt aufweisen, der niedriger als der erste Schmelzpunkt ist.

10. Das Schlaufenelement nach Anspruch 9, wobei der Stapelfaservliesstoff der zweiten Schlaufenschicht (12) in einem Temperaturbereich zwischen dem ersten Schmelzpunkt und dem zweiten Schmelzpunkt verschmolzen ist.

11. Das Schlaufenelement nach einem der Ansprüche 1 bis 10, wobei der Stapelfaservliesstoff der ersten Schlaufenschicht (11), der zweiten Schlaufenschicht (12) und der Basisschicht (13) jeweils Mantel-Kern-Fasern aufweist, die Polypropylen-Kerne und Polyethylen-Mäntel aufweisen.

12. Das Schlaufenelement nach einem der Ansprüche 1 bis 11, wobei die 135°-Schälfestigkeit, gemessen nach JTM-1343, des Schlaufenelements (1) nach einer Wiederholung des In-Eingriff-Bringens und des Außer-Eingriff-Bringens des Schlaufenelements (1) mit/von den Flächenbefestigungsmittelhaken, die vorstehende Teile aufweisen und zum Eingriff in das Schlaufenelement (1) fähig sind, 0,8 N/25,4 mm oder mehr und 10 N/25,4 mm oder weniger beträgt und die 135°-Schälfestigkeit des Schlaufenelements nach zehn Wiederholungen 0,4 N/25,4 mm oder mehr und 10 N/25,4 mm oder weniger beträgt.

13. Eine Windel, aufweisend das Schlaufenelement (1), das in einem der Ansprüche 1 bis 12 beschrieben ist.

## Revendications

1. Élément à boucles (1) pour une fixation de surface, l'élément (1) comprenant une première couche de boucles (11), une deuxième couche de boucles (12) et une couche de base (13) disposées dans cet ordre,
la première couche de boucles (11), la deuxième couche de boucles (12) et la couche de base (13) contenant un non-tissé en fibres discontinues, et
la première couche de boucles (11) et la deuxième couche de boucles (12) ayant des masses volumiques différentes, dans lequel la deuxième couche de boucles (12) a une masse volumique qui est supérieure à la masse volumique de la première couche de boucles (11) et inférieure à la masse volumique de la couche de base (13).

2. Élément à boucles selon la revendication 1, dans lequel
la première couche de boucles (11) a une masse volumique allant de 10 kg/m³ à 80 kg/m³,
la deuxième couche de boucles (12) a une masse volumique allant de 30 kg/m³ à 120 kg/m³, et
la couche de base (13) a une masse volumique allant de 100 kg/m³ à 300 kg/m³.

3. Élément à boucles selon la revendication 1, dans lequel la première couche de boucles (11) et la deuxième couche de boucles (12) ont des degrés de pliabilité différents.

4. Élément à boucles selon la revendication 3, dans lequel la première couche de boucles (11) est plus pliable que la deuxième couche de boucles (12).

5. Élément à boucles selon la revendication 4, dans lequel
la première couche de boucles (11) a une pliabilité telle que mesurée selon un procédé en porte-à-faux JIS L 1096 allant de 1 mm/25 mm de largeur à 40 mm/25 mm de largeur, et
la deuxième couche de boucles (12) a une pliabilité telle que mesurée selon un procédé en porte-à-faux allant de 20 mm/25 mm de largeur à 150 mm/25 mm de largeur.

6. Élément à boucles selon l'une quelconque des revendications 1 à 5, dans lequel
le rapport (B/A) de la densité linéaire moyenne B des fibres de la première couche de boucles (11) à la densité linéaire moyenne A des fibres dans la couche de base est de 1,5 à 30, et
le rapport (C/A) de la densité linéaire moyenne C des fibres de la première couche de boucles (11) à la densité linéaire moyenne A des fibres dans la couche de base est de 1,5 à 30.

7. Élément à boucles selon l'une quelconque des revendications 1 à 6, dans lequel
la première couche de boucles (11) a une épaisseur allant de 80 µm à 700 µm,
la deuxième couche de boucles (12) a une épaisseur allant de 200 µm à 1000 µm, et
la couche de base (13) a une épaisseur allant de 15 µm à 120 pm.

8. Élément à boucles selon l'une quelconque des revendications 1 à 7, dans lequel le non-tissé en fibres discontinues de la deuxième couche de boucles (12) est fondu, et le non-tissé en fibres discontinues de la couche de base (13) est calandré.

9. Élément à boucles selon l'une quelconque des revendications 1 à 8, dans lequel le non-tissé en fibres discontinues de la deuxième couche de boucles (12) inclut des fibres gaine-noyau comprenant un noyau ayant un premier point de fusion et une gaine ayant un deuxième point de fusion inférieur au premier point de fusion.

10. Élément à boucles selon la revendication 9, dans lequel le non-tissé en fibres discontinues de la deuxième couche de boucles (12) est fondu dans une plage de température entre le premier point de fusion et le deuxième point de fusion.

11. Élément à boucles selon l'une quelconque des revendications 1 à 10, dans lequel le non-tissé de fibres discontinues de la première couche de boucles (11), la deuxième couche de boucles (12) et la couche de base (13) incluent chacun des fibres gaine-noyau comprenant des noyaux de polypropylène et des gaines de polyéthylène.

12. Élément à boucles selon l'une quelconque des revendications 1 à 11, dans lequel la résistance au pelage à 135° mesurée selon JTM-1343 de l'élément à boucles (1) après une répétition de mise en prise et de désengagement de l'élément à boucles (1) avec des crochets de fixation de surface comprenant des parties en saillie et étant capable de venir en prise avec l'élément à boucles (1) est non inférieure à 0,8 N/25,4 mm et non supérieure à 10 N/25,4 mm et la résistance au pelage à 135° de l'élément à boucles après dix répétitions est non inférieure à 0,4 N/25,4 mm et non supérieure à 10 N/25,4 mm.

13. Couche comprenant l'élément à boucles (1) décrit selon l'une quelconque des revendications 1 à 12.
